# EUROPEAN PATENT APPLICATION

(11) **EP 1 835 283 A1**
(43) Date of publication of application: **19.09.2007**
(21) Application number: 05814510.3
(22) Date of filing: 07.12.2005
(51) Int. Cl.: G01N 33/53, C12Q 1/68, G01N 5/02, G01N 21/27, G01N 21/64, G01N 33/483, G01N 33/543

(54) **IMMUNE SYSTEM MALFUNCTION DISEASE DIAGNOSIS SUPPORTING METHOD AND DIAGNOSIS SUPPORT INFORMATION OUTPUT DEVICE**

(30) Priority: 10.12.2004 JP 2004357503
(71) Applicant: SYSMEX CORPORATION, Kobe-shi, Hyogo 651-0073 (JP)
(72) Inventor: KITAJIMA, Isao, Toyama-shi, Toyama 939-2719 (JP); YAMANE, Hiroshi, c/o Sysmex Corporation, Kobe-shi, Hyogo 6510073 (JP); YOSHIDA, Tomokazu, c/o Sysmex Corporation, Kobe-shi, Hyogo 6510073 (JP); HASUI, Yasushi, c/o Sysmex Corporation, Kobe-shi, Hyogo 6510073 (JP); TAKEDA, Kazuhiko, c/o Sysmex Corporation, Kobe-shi, Hyogo 6510073 (JP)
(74) Representative: HOFFMANN EITLE
(86) International application number: PCT/JP2005/022453
(87) International publication number: WO 2006/062127

(57) **Abstract**

The invention provides a supporting method for diagnosing immune system malfunction disease and a diagnosis support information output device in order to appropriately treat an immune system malfunction disease such as SIRS whose symptom may drastically change. The supporting method and the device can suggest an appropriate treatment method with consideration of time lag from sampling for diagnosis through determination of a treatment method to the treatment start. By measuring the activated transcriptional factor specific to inflammatory cytokine or anti-inflammatory cytokine in a biological sample, diagnosis support information about the progress of inflammation in the future is outputted.

## Description

### FIELD OF THE INVENTION

The present invention relates to a diagnosis supporting method concerning immune system malfunction diseases including Systemic Inflammatory Response Syndrome (SIRS) such as sepsis, and Compensatory Anti-inflammatory Response Syndrome (CARS), and a diagnosis support information output device, and more particularly to a diagnosis supporting method for outputting diagnosis support information relating to symptoms that are supposed to occur in a few hours by predicting expression of a cytokine as a causal factor in its transcription phase, and a diagnosis support information output device.

### BACKGROUND ART

Sepsis primarily refers to a condition that an infectious disease is developed to a systemic symptom. In an initial phase of sepsis, there are observed a hyperthermia, a hyperpnea, a relatively warm skin, a relatively low blood pressure, a fast heart rate condition with tachycardia, an increased heart rate, and a lowered peripheral vessel resistance. If a suitable treatment is not conducted in this phase, breathing disorder or disorders relating to the kidney, liver, blood, or a like organ may be developed. Various treatments are proposed for the sepsis, including removal of an infectious cause, administration of antibiotics, control of lungs, kidney, and liver, and control of DIC. If a suitable treatment is not conducted, various serious organ failures (multiple organ failure) may be involved, which may cause death. Although intensive therapeutic management has been developed, the death rate of serious sepsis patients is as high as 30 to 50%.

It is reported that patients with the Systemic Inflammatory Response Syndrome (SIRS) including sepsis has a low survival rate, and a high ICU occupancy rate (70%) because proper checkup and treatment depending on a diagnosis concerning severity of pathological condition or a change in pathological condition have not been established.

SIRS was proposed by Dr. Bernard et al. in 1992. Patients with malfunction concerning two or more items among four items representing pathological conditions i.e. the body temperature, the heart rate, the breathing rate, and the number of leukocytes in peripheral blood are diagnosed as SIRS. SIRS is a non-specific systemic inflammatory response invoked by various endogenous mediators, irrespective of the kinds of invasions (surgery, serious external injury, heat injury, infection, etc.), and also includes pathological conditions such as non-infectious, non-specific systemic inflammatory responses, in addition to sepsis.

In the current medical standard, empirically-based treatments and measures based on symptoms have been carried out, as a treatment for SIRS. Specifically, treatments such as a high dose of steroid or a high dose of anti-inflammatory agent are conducted. The symptoms of SIRS such as a hyperthermia or a hyperpnea are caused by a condition (immune hyperactive phase) that an inflammatory response by an inflammatory cytokine is increased. However, a symptom analogous to those of SIRS may also be caused by an anti-inflammatory hyper-responsive condition (immune suppressive phase) due to an anti-inflammatory cytokine. Accordingly, misdiagnosis of the symptom may lead to a treatment whose efficacy is opposite to that of a suitable treatment. For instance, long-term use of steroid or use of an immunosuppressant may develop organ dysfunction if the patient has low immunity, which may cause death in a worst case.

The reason why all the clinical tests conducted by Dr. Bone et al. in 1996 concerning a treatment method for suppressing inflammatory cytokines with respect to sepsis patients ended in failure is presumably because of a physiological activity, within the living body, of keeping a balance between inflammatory response and anti-inflammatory response. The failure leads to an insight that long-term dose of an immunosuppressant may be hazardous.

It is known that many of the diseases resulting from immune system malfunctions involve complex changes or drastic changes in pathological condition resulting from physiologically active substances and cytokines secreted from mediated cells, such as blood neutrophils, lymphocytes, or macrophages. Accordingly, it is very complicated and difficult to select a suitable drug or fix a treatment schedule such as dosage timing. In view of this, serious patients are monitored in ICU to cope with a drastic change in pathological condition. Despite the monitoring, however, this may end in a belated treatment, not to mention a cause of a high ICU occupancy rate.

In view of the above, patent document 1 i.e. Japanese PCT Unexamined Patent Publication (tokuhyo) No. Hei 10-500210 proposes, as a method for identifying and monitoring patients in danger of systemic inflammatory condition, the method comprising: measuring a physiological parameter selected with respect to individual patients; obtaining a test profile on mediator versus related response concerning systemic conditions of the individual patients; and comparing the test profile with a reference profile. Examples of the physiological parameter are physical test result, vital sign, measurement value based on blood dynamics, clinical laboratory test result, concentration of mediator concerning acute inflammatory response, coagulation measurement value on platelet and granulocyte, and endotoxic level. Specifically, leukotriene, prostaglandin, cytokine, platelet activated factor, and neutrophil elastase may be measured by ELISA, complement component C3α may be measured by radio immuno assay, toxic level in plasma may be measured, or coagulation of granular leukocyte may be measured.

All the above-mentioned physiological parameters are obtained by measuring proteins or proliferative factors contained in samples such as blood obtained from the patients. Even if a protein, as a targeted substance for measurement, is a secretion from targeted cells, and represents a condition of the cells at the point of time when the sample was obtained, it is not guaranteed that the cell condition can be retained thereafter. Pathological conditions i.e. expression of a cytokine causing symptoms are drastically changed with time. Therefore, it is difficult to establish a treatment method for immune system malfunction diseases. Since a condition concerning the cytokine expression may be changed in a time from the point of time when the sample was obtained until the measurement result is obtained, for instance, even if significant expression of a cytokine is observed as a result of measurement on the cytokine expression in an obtained sample, it is not always proper to perform a treatment of suppressing the cytokine.

### DISCLOSURE OF THE INVENTION

In view of the above, it is an object of the invention to provide an immune system malfunction disease diagnosis supporting method that enables to suggest proper diagnosis support information in treating immune system malfunction diseases whose symptoms may be drastically changed. It is another object of the invention to provide a diagnosis support information output device that enables to output proper diagnosis support information.

A supporting method of the invention comprises: measuring an amount of an activated transcriptional factor specific to a cytokine selected from the group consisting of inflammatory cytokines and anti-inflammatory cytokines in a biological sample obtained from a patient; and outputting diagnosis support information including information relating to progress condition of inflammations in the future, based on the measured amount of the activated transcriptional factor.

The information relating to progress condition of inflammation may include information selected from the group consisting of information as to whether an inflammatory response will be increased, and information as to whether an anti-inflammatory response will be increased. Alternatively, the information relating to progress condition of inflammation may include cytokine expression information selected from the group consisting of information as to whether the expression level of an inflammatory cytokine is increased, and information as to whether the expression level of an anti-inflammatory cytokine is increased.

The diagnosis support information may further comprise information relating to a treatment method of the disease. The treatment method is preferably selected from the group consisting of administration of an anti-inflammatory agent, exchange of plasma, and administration of granulocyte colony-stimulating factor.

The preferred biological sample comprises cells taken out from the patient.

The measuring step is preferably performed with use of a measuring method which does not require more than one hour, more preferably, more than 30 minutes, for a measurement start until a measurement result is obtained. Preferably, the measuring method is one selected from the group consisting of surface plasmon resonance method, quartz crystal microbalance method, and fluorescence correlation spectroscopy method.

The measuring step may include a step of measuring two or more kinds of activated transcriptional factors.

The inventive supporting method for diagnosing immune system malfunction disease is suitable for diagnosing systemic inflammatory response syndrome (SIRS).

The invention further provides a diagnosis support information output device concerning immune system malfunction diseases. The device comprises an information acquiring means for acquiring information relating to transcriptional factors obtained by measuring an amount of an activated transcriptional factor specific to a cytokine selected from the group consisting of inflammatory cytokines and anti-inflammatory cytokines, in a biological sample obtained from a patient; a diagnosis support information outputting means for outputting diagnosis support information including information relating to progress conditions of inflammations in the future, based on the acquired regulator information.

The information relating to progress condition of inflammation may be selected from the group consisting of information as to whether an inflammatory response will be increased, and information as to whether an anti-inflammatory response will be increased. Alternatively, the information relating to progress condition of inflammation may include cytokine expression information selected from the group consisting of information as to whether the expression level of an inflammatory cytokine is increased, and information as to whether the expression level of an anti-inflammatory cytokine is increased.

Preferably, the diagnosis support information comprises information relating to a treatment method of the disease. The preferable treatment method is selected from the group consisting of administration of an anti-inflammatory agent, exchange of plasma, and administration of granulocyte colony-stimulating factor.

Preferably, the diagnosis support information outputting means includes a storing means for storing information relating to a transcriptional factor specific to an inflammatory cytokine, and a predicting means for predicting whether the expression level of the inflammatory cytokine will be increased, based on the information stored in the storing means and the transcriptional factor information acquired by the information acquiring means. Diagnosis support information outputted from the diagnosis support information outputting means is based on a prediction result by the predicting means. The storing means may further store information relating to a transcriptional factor specific to an anti-inflammatory cytokine.

Alternatively, the diagnosis support information outputting means may include a storing means for storing information as to whether an inflammatory response is increased, and a predicting means for predicting whether an inflammatory response will be increased, based on the information stored in the storing means and the transcriptional factor information acquired by the information acquiring means. Diagnosis support information outputted from the diagnosis support information outputting means is based on a prediction result by the predicting means. The storing means may further store information as to whether an anti-inflammatory response will be increased.

The term "Activated transcriptional factor" in the specification is used for differentiating from a transcriptional factor which exist as a complex in cytoplasm. The activated transcriptional factor is a factor released from the complex and transported into nucleus in order to transcript a gene and express a cytokine encoded by the gene.

With use of the inventive immune system malfunction disease diagnosis supporting method, the diagnosis support information can be outputted, based on the activated transcriptional factor which regulates a transcription phase before expression of the cytokine causing symptoms. The diagnosis support information includes the information relating to progress conditions of inflammations, which are supposed to occur in the future before secretion of the cytokine.

The inventive diagnosis support information output device outputs the diagnosis support information relating to inflammatory conditions which are supposed to occur in a few hours, based on samples obtained from patients with immune system malfunction diseases, whose symptoms are changed with time. With use of the device, a suitable treatment can be conducted, and precautionary measures can be taken before a serious symptom occurs.

### BRIEF DESCRIPTION OF THE DRAWINGS

FIG. 1 is a DNA sequence diagram showing sites to which respective transcriptional factors bind.
FIG. 2 is a diagram showing relations between transcriptional factors, and cytokines involved in the transcriptional factors.
FIG. 3 is a diagram for describing a measurement principle by a surface plasmon resonance (SPR) method.
FIG. 4 is a diagram for describing a measurement principle by a quartz crystal microbalance (QCM) method.
FIG. 5 is a diagram for describing a measurement principle by a fluorescence correlation spectroscopy (FCS) method.
FIG. 6 is a diagram for describing an example as to how a SIRS condition is changed to a CARS condition.
FIG. 7 is a block diagram showing a hardware configuration of an immune system malfunction disease diagnosis support system provided with a diagnosis support information output device according to an embodiment of the invention.
FIG. 8 is a flowchart showing an embodiment of a diagnosis support information output process.
FIG. 9 is a micrograph (400 magnifications) showing an immunostaining result in the case where HeLa cells was not treated by lipopolysaccharide.
FIG. 10 is a micrograph (400 magnifications) showing an immunostaining result in the case where HeLa cells was treated by lipopolysaccharide.
FIG. 11 is a photo showing a result of gel shift assay.
FIGS. 12(a) and 12(b) are sequence diagrams of nucleotide probes used in examples.
FIG. 13 is a graph showing measurement results as to whether HeLa cells bind to the probes.
FIG. 14 is a photo showing a result of gel shift assay as to whether lymphocytes of healthy individuals bind to the probes.
FIG. 15 is a graph on SPR measurement results showing a relation of NFκB concentration versus amount of NFκB-probe complex.
FiG.16 is a graph on FCS measurement results showing a relation of NFκB concentration versus amount of NFκB-probe complex.
FIG.17 is a graph on QCM measurement results showing a relation of NFκB concentration versus amount of NFκB-probe complex.
FIG.18 is a graph on SPR measurement results showing a relation of SP-1 concentration versus amount of SP-1-probe complex.
FIG.19 is a graph on SPR measurement results showing a relation of AP-1 concentration versus amount of AP-1-probe complex.
FIG.20 is an electrophoresis photograph showing an analysis of expression results of IL6 mRNA and G-CSF mRNA.
FIG.21 is a photograph showing a result of gel shift assay of NFκB.

### BEST MODE FOR CARRYING OUT THE INVENTION

### [supporting method for diagnosing immune system malfunction disease]

The inventive supporting method for diagnosing immune system malfunction disease comprises: measuring an amount of an activated transcriptional factor specific to a cytokine selected from the group consisting of inflammatory cytokines and anti-inflammatory cytokines in a biological sample obtained from a patient; and outputting diagnosis support information including information relating to progress condition of inflammations in the future, based on the measured amount of the activated transcriptional factor.

Examples of the diagnosing immune system malfunction diseases applied by the inventive supporting method include Systemic Inflammatory Response Syndrome (SIRS) such as sepsis, and Compensatory Anti-inflammatory Response Syndrome (CARS). Particularly, the supporting method of the invention is suitable for supporting diagnosis of SIRS.

The biological sample is a sample containing cells obtained from a patient. Examples of the biological samples include blood, urine, bronchiole alveolus cleaning fluid, saliva, phlegm, cerebrospinal fluid, joint fluid, infiltrating fluid, and so on. A preferable sample for measurement is nuclear extract of cells obtained from the biological sample, because the activated transcriptional factor to be measured is an activated transcriptional factor which is associated with transcription of the cytokine scheduled to be expressed soon and exists in nucleus. An inactive transcriptional factor usually exists as a complex in cytoplasm. Therefore, it is difficult to measure an amount of the transcriptional factor by a method utilizing interaction between the transcriptional factor and biological molecules such as antibody. On the other hand, since an activated transcriptional factor is released from the complex in response to stimulation occurred and can be differentiated from the transcriptional factor existing as a complex, the amount of the activated transcriptional factor can be measured by a method utilizing interaction between the activated transcriptional factor and a biological molecule such as antibody.

A sample containing the nuclear extract is prepared from a sample obtained from a patient. The preparation method, for example, comprises treating a cell with hypotonic solution to expand the cell, breaking the cell membrane using homogenizer or syringe, isolating pellet containing nucleus of the cell by centrifuging, treating the pellet with hypertonic solution or detergent to extract nucleoprotein from the pellet, following by centrifugation, and saving the supernatant. The supernatant contains protein extract of the nucleus. But the preparation method is not limited to the above.

The activated transcriptional factor regulates transcription activity by binding a specific enhancer sequence whose function is to increase an expression level of a gene encoding the targeted inflammatory cytokine or anti-inflammatory cytokine. The activated transcriptional factor to be measured is suitably selected depending on a kind of the targeted inflammatory cytokine or anti-inflammatory cytokine. Since a transcriptional factor has a DNA binding domain (DBD) and a transcription activating domain (TAD), the transcriptional factor is able to recognize a specific sequence and bind it. A transcriptional factor promotes a transcription initiation complex with mediator, or stabilizes the complex, by binding to enhancer.

Examples of the inflammatory cytokine include Tumor Necrosis factor (TNFα), interleukin 1, interleukin 2, interleukin 6, interleukin 12, interleukin 18, Platelet Activating factor (PAF), G-CSF, GM-CSF, interferon γ.

Examples of the anti-inflammatory cytokine include interleukin 4, interleukin 10, interleukin 13, interleukin 1 receptor, soluble TNF receptor, TGF β.

Expressions of these cytokines are controlled in their transcription phase under respective specific regions on their DNA (specific sequences of DNA). An activated transcriptional factor binds to a transcription regulatory region on DNA which regulates expression of cytokine. Particularly, an activated transcriptional factor and RNA polymerase bind enhancer and promoter, respectively, and thereby transcription is activated and initiated. The produced mRNA is translated to synthesize the cytokine coded by the mRNA.

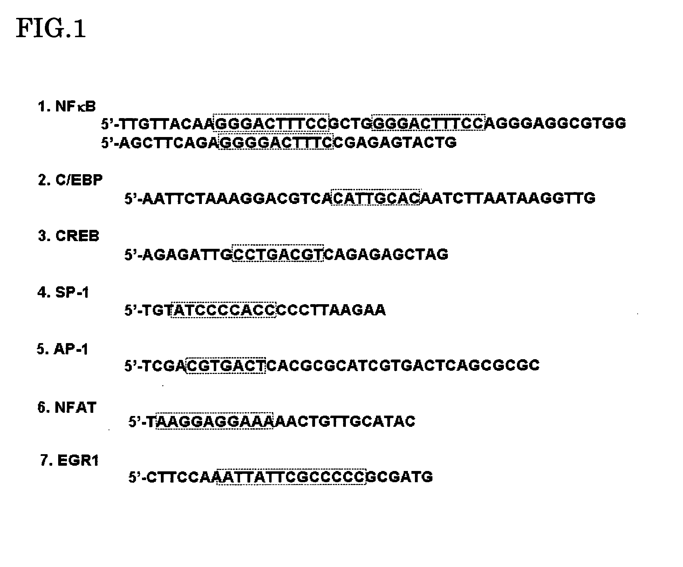

There are about 20 kinds of transcriptional factors relating to expression of cytokines. Each of the transcriptional factors binds to a specific sequence of DNA. FiG.1 shows examples of sequences to which some transcriptional factors bind specifically. In FIG.1, sequence portions to be bound by respective transcriptional factors are bordered by dot line.

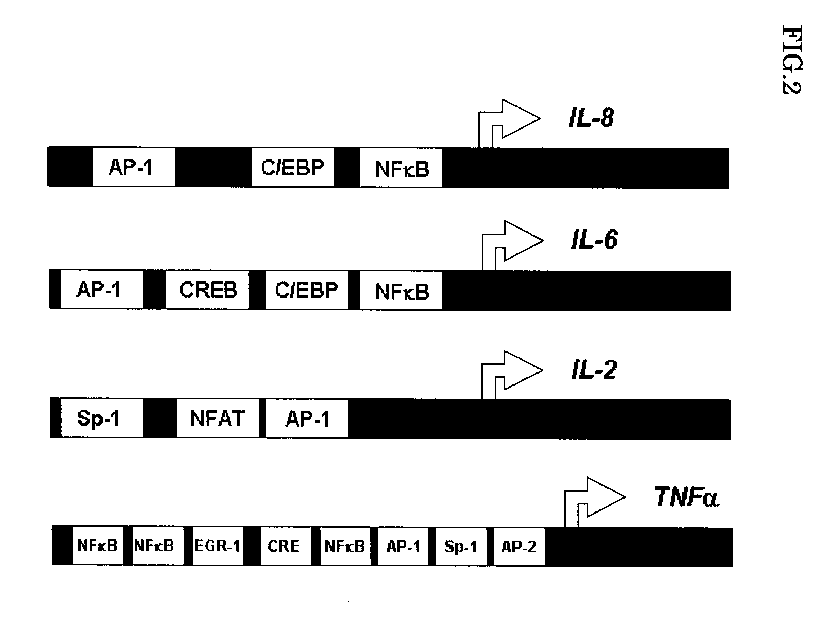

A transcriptional factor regulates transcription of DNA coding a cytokine. Transcriptional factors differ from each other by sorts of the cytokine to be expressed. Usually, two or more transcriptional factors cooperate to regulate expression of one kind of cytokine. FIG.2 shows examples: an expression of interleukin 8 (IL8) is associated with transcriptional factors such as AP-1, C/EBP and NFκB; an expression of interleukin 6 (IL6) is associated with transcriptional factors such as AP-1, CREB, C/EBP and NFκB; an expression of interleukin 2 (IL2) is associated with transcriptional factors such as NFAT and AP-1; and an expression of TNFα is associated with transcriptional factors such as NFκB, BRG1, CRE, AP-1, SP1 and AP-2. When TNFα is contemplated to be expressed, an amount of NFκB transferred into nucleus is supposed to be larger, comparing when a cytokine such as IL8 and IL6 are contemplated to be expressed. This is because there are some binding sites of NFκB in transcription regulatory region of TNFα, while there is one binding site of NFκB in respective transcription regulatory regions of IL8 and IL6. An expression of IL4 is associated with transcriptional factors such as AP-1 and C/EBP. An expression of IL10 is associated with transcriptional factors such as RE1, CRE3, CRE4, TATA, C/EBP1, C/EBP3, and C/EBP5.

Any activated transcriptional factor capable of predicting the expression level of an inflammatory cytokine or an anti-inflammatory cytokine can be used. In the case where expression of inflammatory cytokines is predicted, one kind of an activated transcriptional factor that is commonly involved in expression of inflammatory cytokines but not involved in expression of anti-inflammatory cytokines may be measured. In the case where expression of anti-inflammatory cytokines is predicted, one kind of an activated transcriptional factor that is commonly involved in expression of anti-inflammatory cytokines but not involved in expression of inflammatory cytokines may be measured. The amounts of plural kinds of activated transcriptional factors may be measured so that expressions of individual cytokines may be predicted based on the amount ratios of the activated transcriptional factors. For instance, cytokines particularly involved in SIRS are IL8, IL6, and TNFα, and the transcriptional factor commonly involved in expression of these cytokines is NFκB. Accordingly, measuring the amount of active NFκB enables to predict whether the expression level of the inflammatory cytokines is increased.

Measurement of the activated transcriptional factor may be preferably conducted by a method with a measurement time of 1 hour or less from a measurement start until a measurement result is obtained. More preferably, the measurement is conducted by a method with a measurement time of 30 minutes or less. If a measuring method requiring a few hours or more is used to obtain a measurement result, a cytokine, which is supposed to be expressed upon lapse of a few hours after transcription start, may be expressed in patients before the measurement result is obtained. In a worse case, a cytokine other than the targeted cytokine may be expressed, which may delay a treatment or lead to an improper treatment.

Preferred examples of the method with a measurement time of 30 minutes or less are a method of detecting nucleotide hybridization, using a nucleotide probe having a specific sequence to which a transcriptional factor binds, and a method of measuring inter-biomolecular interaction. Specific examples of the method are surface plasmon resonance (SPR) method, quartz crystal microbalance (QCM) method, fluorescence correlation spectroscopy (FCS) method, and dual polarization interferonmeter-SPR.

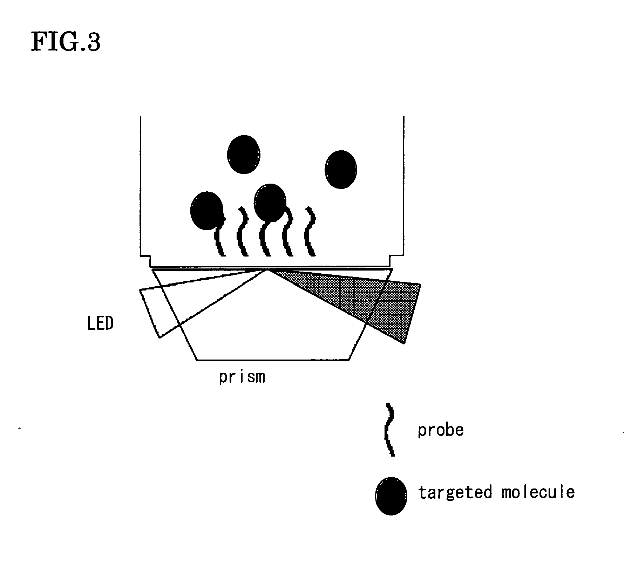

The SPR method is a method, as shown in FIG. 3, using a surface plasmon resonance phenomenon, which is generated by reflection of light which is incident onto a metallic membrane (gold or silver film layer) coated on a prism surface with a specific angle, and which goes out therefrom by stimulation of electrons on the metal surface. Since the surface plasmon resonance and a resonant angle depend on a reflective index, presence or absence of a nucleotide hybrid on the surface of the metallic membrane, i.e., the amount of a transcriptional factor that binds to the DNA probe can be measured.

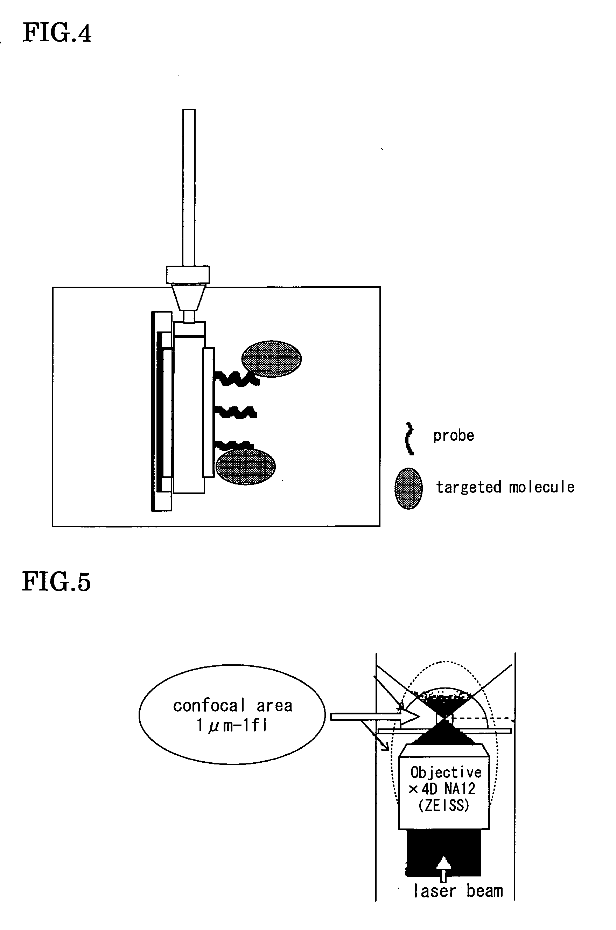

The QCM method is a method, as shown in FIG. 4, for detecting a change in mass of a metallic membrane resulting from binding of a transcriptional factor contained in a sample to a nucleotide probe, in terms of a change in the frequency, using a quartz crystal covered with the metallic membrane implanted with the nucleotide probe.

The FCS method is a method, as shown in FIG. 5, comprising: allowing a mixture of a fluorescence-labeled nucleotide probe and a sample to be measured to pass through a confocal area; and separating a transcriptional factor-probe complex from an unreacted fluorescent molecule (probe itself) by the number of pulses with which one molecule is irradiated, utilizing a difference in time required for the transcriptional factor-probe complex and the probe itself to pass the confocal area due to the molecular size difference thereof.

The nucleotide probe to be used in the aforementioned methods may be an oligonucleotide probe having a base sequence to which the transcriptional factor to be detected specifically binds. As the nucleotides probe, a wild type probe whose entire sequence exists in the nature, or an artificially synthesized probe may be used. The nucleotide probe to be used may be of a single kind, or of plural kinds of the number corresponding to the number of transcriptional factors involved in cytokines whose expression is to be predicted. Preferably, the nucleotide probes of the number corresponding to the number of all the possible transcriptional factors may be provided. This is preferred because all the possible cytokines to be expressed can be predicted by detecting and quantitatively determining the plural kinds of transcriptional factors by a one-time measurement.

The method for measuring inter-biomolecular interaction has a detection sensitivity of 10 nM, and can quantitatively determine one sample in about 10 minutes. With use of the method, a time required from a measurement start until a measurement result is obtained, including a time required for obtaining cellular specimen from a patient, and preparing a sample to be measured, is one hour or less. Accordingly, cytokines which are supposed to be expressed within a few hours from the point of time when the biological sample was obtained from the patient can be identified. This is advantageous in predicting whether a symptom which is supposed to occur within a few hours is an anti-inflammatory response or an inflammatory response, and in predicting the kind of cytokine which is supposed to be expressed, and the symptom which is supposed to occur. As compared with this method, prediction by ELISA or a gel shift assay takes 3 to 6 hours. According to the conventional methods, it is difficult to select a suitable treatment because it is highly likely that a cytokine other than the cytokine whose expression is intended to be predicted may be expressed before a prediction result is obtained. Thus, use of the speedy measuring method proposed in the embodiment as mentioned above is advantageous in selecting a suitable treatment and prescription based on the prediction.

Diagnosis support information is outputted based on the amount of the measured activated transcriptional factor. In the embodiment, the diagnosis support information is information relating to progress conditions of inflammations in the future, and may include information selected from the group consisting of information as to whether an inflammatory response is increased, and information as to whether an anti-inflammatory response is increased, or may include cytokine expression-related information selected from the group consisting of information as to whether the expression level of an inflammatory cytokine is increased, and information as to whether the expression level of an anti-inflammatory cytokine is increased.

Knowing the amount of the activated transcriptional factor involved in secretion of a targeted cytokine (inflammatory cytokine or anti-inflammatory cytokine) enables to predict whether the inflammatory cytokine or the anti-inflammatory cytokine to be expressed will be increased, and resultantly enables to predict progress conditions of inflammations in the future as to whether an inflammatory response will be increased or an anti-inflammatory response will be increased. Thus, the prediction results can be outputted as diagnosis support information.

Particularly, adopting the measuring method requiring only 30 minutes or less from a measurement start until a measurement result is obtained enables to predict the cytokine which is supposed to be expressed within a few hours from the point of time when a sample was taken out from a patient. Accordingly, prediction can be made as to whether the symptom which is supposed to occur within a few hours is an increase of an anti-inflammatory response or an increase of an inflammatory response.

The diagnosis support information may include information relating to treatment methods to be selected, and information relating to adequacy/inadequacy of the treatment methods (information relating to adequacy/inadequacy on administration of an anti-inflammatory agent, information relating to adequacy/inadequacy on exchange of plasma, and information relating to adequacy/inadequacy on administration of a granulocyte colony-stimulating factor). Adopting the rapid measuring method enables to predict whether a symptom which is supposed to occur within a few hours is an anti-inflammatory response or an inflammatory response, and further enables to predict the kind of cytokine which is supposed to be expressed, and the symptom which is supposed to occur. Thus, selection of a suitable treatment and prescription based on the prediction can be outputted as the diagnosis support information.

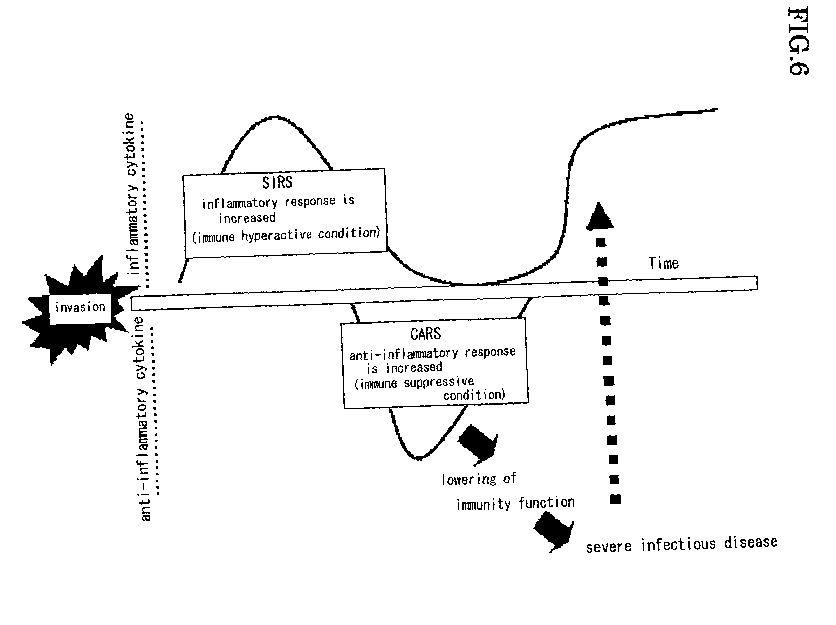

For instance, as shown in FIG. 6, in a late phase of SIRS, the expression level of an inflammatory cytokine is decreased, and the expression level of an anti-inflammatory cytokine is increased, which corresponds to a condition in a phase of CARS. In such a condition, if a treatment based on a conventional diagnosis by measurement and detection of cytokine is conducted, an anti-inflammatory agent may be administered. On the other hand, with use of the method of the embodiment, information can be obtained, based on the amount of an activated transcriptional factor, as to whether the measurement result indicates a first phase of SIRS when expression of an inflammatory cytokine is increased, or a late phase of SIRS when expression of an inflammatory cytokine is decreased, or a phase of CARS when expression of an anti-inflammatory cytokine is increased. Accordingly, based on the diagnosis support information outputted by the inventive method, the user can select a suitable treatment method such as administering an anti-inflammatory agent in a phase of SIRS when expression of an inflammatory cytokine is increased; and exchanging plasma or administering a granulocyte colony-stimulating factor in a phase of CARS when expression of an anti-inflammatory cytokine is increased.

### [Diagnosis Support Information Output Device Concerning Immune System Malfunction Diseases]

The diagnosis support information output device concerning immune system malfunction diseases according to the invention comprises an information acquiring means for acquiring information relating to transcription regulators, which is obtained by measuring the amount of an active transcription regulator specific to a cytokine which is contained in a biological sample obtained from a patient, and is selected from the group consisting of inflammatory cytokines and anti-inflammatory cytokines; and a diagnosis support information outputting means for outputting diagnosis support information including information relating to progress conditions of inflammations in the future, based on the acquired transcriptional factor information.

The transcriptional factor information may be the amount of the activated transcriptional factor to be measured, or a digital signal obtained from a measuring device for measuring the amount, e.g. a digital signal corresponding to an optical intensity obtained before conversion into the amount such as a fluorescent intensity or a reflectance, a radioactive intensity, or the frequency.

The information relating to progress conditions of inflammations in the future may be information selected from the group consisting of information as to whether an inflammatory response is increased, and information as to whether an anti-inflammatory response is increased, or may include information relating to cytokine expression, which is selected from the group consisting of information as to whether the expression level of an inflammatory cytokine is increased, and information as to whether the expression level of an anti-inflammatory cytokine is increased.

Preferably, the diagnosis support information may further include information relating to treatment methods, in addition to the information relating to progress conditions of inflammations in the future. Preferably, the treatment methods may be selected from the group consisting of administration of anti-inflammatory agent, exchange of plasma, and administration of granulocyte colony stimulating factor.

Preferably, the diagnosis support information outputting means may include a storing means for storing information relating to transcriptional factors specific to inflammatory cytokines; and predicting means for predicting whether the expression level of the inflammatory cytokine will be increased, based on the transcriptional factor information acquired by the information acquiring means, and the information stored in the storing means, wherein diagnosis support information is outputted based on a prediction result by the predicting means. The storing means may further store information relating to transcriptional factors specific to anti-inflammatory cytokines.

Alternatively, the diagnosis support information outputting means may include a storing means for storing information as to whether the inflammatory response is increased; and a predicting means for predicting whether the expression level of the inflammatory cytokine will be increased, based on the transcriptional factor information acquired by the information acquiring means, and the information stored in the storing means, wherein diagnosis support information is outputted based on a prediction result by the predicting means. The storing means may further store information as to whether the anti-inflammatory response is increased.

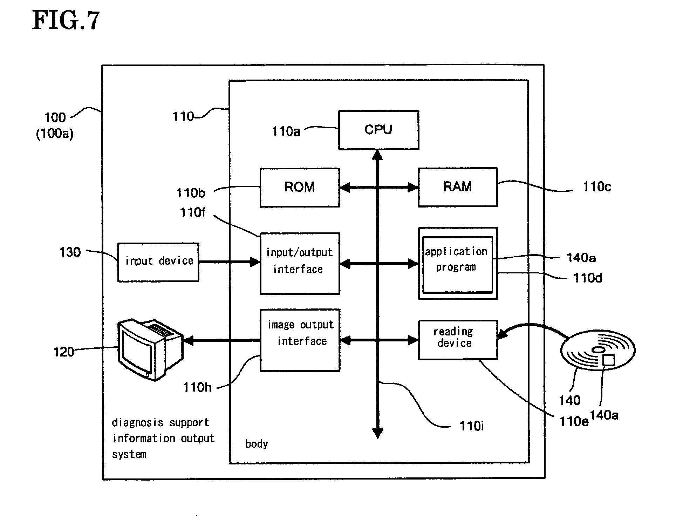

An embodiment of the diagnosis support information output device of the invention is described referring to a hardware configuration example shown in FIG. 7.

A system 100 provided with the diagnosis support information output device of the embodiment includes a computer 100a provided with a computer main body 110, a display 120, and an input device 130 as primary components. The computer main body 110 includes a CPU 110a, an ROM 110b, an RAM 110c, a hard disk 110d, a reading device 110e, an input/output interface 110f, a communication interface 110g, and an image output interface 110h, as primary components. The CPU 110a, the ROM 110b, the RAM 110c, the hard disk 110d, the reading device 110e, the input/output interface 110f, and the image output interface 110h are interactively connected with each other by a bus 110i for data communication.

In the diagnosis support information output system having the configuration as shown in FIG. 7, the input/output interface 110f corresponds to the information acquiring means, and the computer main body 110 corresponds to the diagnosis support information outputting means. Also, the storing means corresponds to the ROM 110b, the RAM 110c, and the hard disk 110d; and the predicting means corresponds to the CPU 110a.

Examples of the input/output interface 110f include a serial interface such as USB, IEEE1394, or RS-232C, a parallel interface such as SCSI, IDE, or IEEE1284, and an analog interface such as a D/A converter or an A/D converter. The input/output interface 110f is connected to the input device 130 provided with a keyboard and a mouse, and is operative to input data to the computer 100a in response to user's manipulation of the input device 130.

The transcriptional factor information including measurement data may be entered by the user via the input device 130. Alternatively, an output section of a measuring device for measuring the amount of an activated transcriptional factor may be connected to the input/output interface 130 to directly input a digital signal obtained by the measurement as the transcriptional factor information.

The CPU 110a as the predicting means is operative to execute a computer program stored in the ROM 110b as the storing means, and a computer program loaded in the RAM 110c. When an application program 140a to be described later is executed by the CPU 110a, the computer 100a is functioned as the diagnosis support system 100. Specifically, the diagnosis support system 100 is operative to predict whether the cytokine to be expressed is an inflammatory cytokine or an anti-inflammatory cytokine, based on the activated transcriptional factor information inputted by the information acquiring means, and to output diagnosis support information based on the prediction result. In the case where the information stored in the storing means is the information relating to transcriptional factors which cause an increase of the inflammatory response or the anti-inflammatory response, the diagnosis support system 100 is operative to predict whether the inflammatory response will be increased or the anti-inflammatory response will be increased, and to output diagnosis support information based on the prediction result.

The storing means stores information necessary for outputting the diagnosis support information based on the transcriptional factor information acquired by the information acquiring means, specifically, information relating to relations between transcriptional factors and inflammatory cytokines or anti-inflammatory cytokines, or information relating to relations between transcriptional factors and cytokines which cause an increase of an inflammatory response or an anti-inflammatory response. The storing means may also store the information relating to treatment methods. These information may be stored at least in any one of the ROM 110b, the RAM 110c, and the hard disk 110d. The information relating to relations between cytokines and transcriptional factors may include e.g. information relating to the kinds of cytokines to be expressed in association with the transcriptional factors, information relating to the expression levels of the cytokines corresponding to the amounts of the transcriptional factors, and respective times from the point of time when the transcriptional factor is expressed to the point of time when the cytokine is expressed.

Examples of the ROM 110b include a mask ROM, a PROM, an EPROM, and an EEPROM. The ROM 110b is recorded with a computer program to be executed by the CPU 110a, data to be used in the execution of the computer program, and the like, in addition to the information relating to the relations between transcriptional factors and cytokines, and the information relating to treatment methods.

Examples of the RAM 110c include an SRAM and a DRAM. The RAM 110c is adapted to read the computer programs recorded in the ROM 110b and the hard disk 110d. The RAM 110c is used as a working area in executing the computer programs by the CPU 110a. The RAM 110c may temporarily store the transcriptional factor information inputted from the input/output interface 110f.

The hard disk 110d is installed with various computer programs to be executed by the CPU 110a, such as an operating system (e.g. an operating system of providing a graphical user interface environment such as the Windows^{®} manufactured and sold by the Microsoft Corporation, U.S.A.), and an application program, in addition to the information relating to relations between cytokines and transcriptional factors. The application program 140a to be described later is also installed in the hard disk 110d.

Examples of the reading device 110e include a flexible disk drive, a CD-ROM drive, and a DVD-ROM drive. The reading device 110e is adapted to read a computer program or data recorded in a portable recording medium 140. The portable recording medium 140 stores the application program 140a for causing the computer 100a to function as the diagnosis support system of the invention. The computer 100a is operative to read the application program 140a of the invention from the portable recording medium 140, and to install the application program 140a in the hard disk 110d.

The application program 140a may be provided by an external device communicatively connected to the computer 100a via an electrical communication line (including wired and wireless communication lines), in place of being provided by the portable recording medium 140. For instance, the application program 140a may be stored in a hard disk of a server computer connected to the user's computer 100a via the Internet so that the user can install the application program 140a in the hard disk 110d by accessing the server computer with use of the computer 100a and by downloading the application program i.e. the computer program.

The image output interface 110h is connected to the display 120 provided with an LCD, a CRT, or a like display device. The image output interface 110h is adapted to output a video signal in accordance with image data provided from the CPU 110a on the display 120. The display 120 displays an image (screen image) in accordance with the inputted video signal.

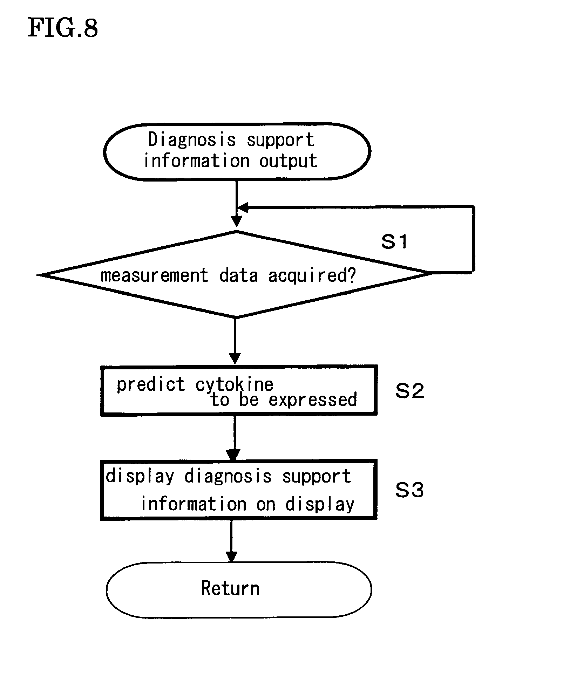

Now, an embodiment of a diagnosis support information output process to be executed by the information outputting means is described referring to FIG.8, which shows a flowchart of a diagnosis support information output process concerning SIRS.

First, the computer 100a acquires measurement data concerning an activated transcriptional factor obtained by measuring a blood sample of a patient (S1). The measurement data is acquired by inputting the measurement data concerning the activated transcriptional factor measured by the measuring device by way of the input device 130. Also, the point of time when the sample was obtained, the time required for measuring the transcriptional factor, and the like are inputted by way of the input device 130. Alternatively, the measurement data concerning the activated transcriptional factor may be acquired from a measuring device connected to an LAN via the input/output interface 110f of the computer 100a by connecting the computer 100a and the measuring device by a network such as the LAN.

Then, the CPU 110a predicts a cytokine to be expressed based on the acquired measurement data (S2). The prediction of the cytokine to be expressed is conducted based on the expression level of the measured active NFκB. NFκB is a transcriptional factor specific to inflammatory cytokines (IL8, IL6, TNFα) involved in SIRS. Accordingly, if NFκB is expressed in a predetermined amount or more, it is possible to predict that any one of the inflammatory cytokines i.e. IL8, IL6, and TNFα will be expressed within a few hours.

Then, the diagnosis support information corresponding to the prediction of the cytokines to be expressed is selected to display the selected diagnosis support information on the display 120 (S3). The diagnosis support information includes a prediction result as to whether the expression level of the inflammatory cytokine will be increased from the point of time when the sample was obtained, and information as to whether a certain treatment method (administration of an anti-inflammatory agent) is suitable.

In this embodiment, prediction of the cytokine to be expressed is made based on the measurement data concerning NFκB. Alternatively, the kind of the cytokine to be expressed may be predicted by increasing the number of kinds of transcriptional factors to be measured. In the case where expression of IL8 is predicted, the kinds and quantitative ratio (such as measurement values and quantitative values) of transcriptional factors are AP1:C/EBP:NFKB=1:1:1. In the case where expression of IL6 is predicted, the kinds and quantitative ratio of transcriptional factors are AP1:C/EBP:NFKB:CREB=1:1:1:1. In the case where expression of IL2 is predicted, the kinds and quantitative ratio of transcriptional factors are AP1:NFAT:Sp1=1:1:1. In the case where expression of TNFα is predicted, the kinds and quantitative ratio of transcriptional factors are AP1:NFrB:Sp1:EGR1:AP2:CRE1=1:3:1:1:1:1. In the case where expression of IL4 is predicted, the kinds and quantitative ratio of transcriptional factors are AP1:C/EBP = 1:2. In the case where expression of IL10 is predicted, the kinds and quantitative ratio of transcriptional factors are CRE1:CRE3:CRE4:TATA:C/EBP1:C/EBP3:C/EPB5=1:1:1:1:1:1:1.

Since these quantitative ratios include measurement errors among individuals, or by sample preprocessing steps, the transcriptional factor amounts include measurement errors of ± about 0.5. For instance, if the kinds and quantitative ratio of transcriptional factors are AP1:C/EBP:NFκB:CREB = 1.2:0.6:0.9:1.3, it is predicted that the cytokine to be expressed is IL6.

### EXAMPLES

### [Preparation method of nuclear extract sample]

### (1) Preparation of nuclear extract sample using HeLa cells

Preparation was conducted according to the method recited in Nucleic Acids Res., published on March 11, 1983, 11(5), pp.1475-1489.

Specifically, a culture of HeLa cells was centrifuged at 4°C, 2,000 rpm for 10 minutes. The harvested HeLa cells were washed with 5 to 10 ml of ice-cold phosphate buffer salt (PBS) solution (centrifuged at 4 °C, 2,000 rpm for 10 minutes). The washed HeLa cells were suspended in 5 to 10 ml of buffer A (final concentration: 10 mM HEPES (pH 7.9), 1.5 mM MgCl₂, 10 mM KC1, 0.5 mM DDT), and the resultant suspension was incubated to stand still for 10 minutes. By stereomicroscopic observation of the suspension, expansion of the cells was confirmed. Then, the suspension was centrifuged at 4 °C, 2,000 rpm for 10 minutes, and the cells were collected. The collected cells were resuspended in 1 to 5 ml of buffer A.

Next, the cells were broken, by using a Dounce homogenizer with 20 to 40 strokes, or by syringing through a 24-gauge needle with 20 to 40 strokes. By stereomicroscopic observation, it was confirmed that cell membranes were broken, and bare nuclei appeared.

Thereafter, the cell homogenate was recovered by centrifuging at 4 °C, 15,000×g for 20 minutes. Then, the cell homogenate was suspended in 1 ml of buffer C (final concentration: 20 mM HEPES (pH 7.9), 25v/v% glycerol, 0.42M NaCl, 1.5 mM MgCl₂, 0.2 mM EDTA, 0.5 mM PMSF, 0.5 mM DDT). Then, the nuclei in the suspension were broken, by using a Dounce homogenizer with 20 to 40 strokes, or by syringing through a 24-gauge needle with 20 to 40 strokes. Then, the suspension was centrifuged at 4 °C, 15,000 x g for 30 minutes, and a supernatant was saved. The supernatant was freezed by liquid nitrogen, and stored at -80 °C.

In the case where desalination is necessary, the suspension is subjected to dialysis in 50 times its volume of buffer D (final concentration: 20 mM HEPES (pH 7.9), 25v/v% glycerol, 0.1M KCl, 0.2mM EDTA, 0.5 mM PMSF, 0.5mM DDT) for 5 hours.

### [Verification on transfer of transcriptional factor into nuclei]

### (1) Microscopic observation on immunostaining]

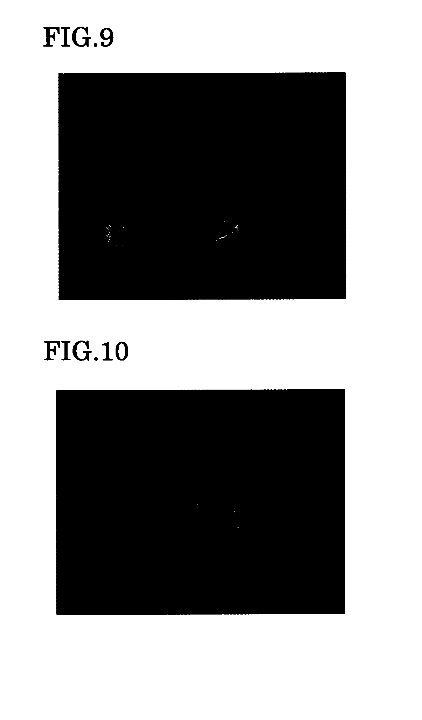

Cultured HeLa cells were reacted with fluorescent-labeled anti-NFκB antibody, and the reaction solution was incubated to stand still for 120 minutes. Thereafter, the resultant was observed through a fluorescent microscope. A micrograph (magnification: 400 times) is shown in FIG. 9. The micrograph shows that the periphery of the cell nuclei was stained with the fluorescence. This shows that NFκB stayed within the cytoplasm when the cells were not stimulated.

Also, cultured HeLa cells were reacted with fluorescent-labeled anti-NFκB antibody by administering 2.5 mg/ml of lipopolysaccharide. After the reaction, the reaction solution was incubated to stand still for 120 minutes. Then, the resultant was observed through a fluorescent microscope. A micrograph (magnification: 400 times) is shown in FIG. 10. The micrograph shows that the interior of the nuclei was stained with the fluorescence. This shows that NFκB was transferred into the nuclei by treatment of lipopolysaccharide.

### (2) Gel shift assay

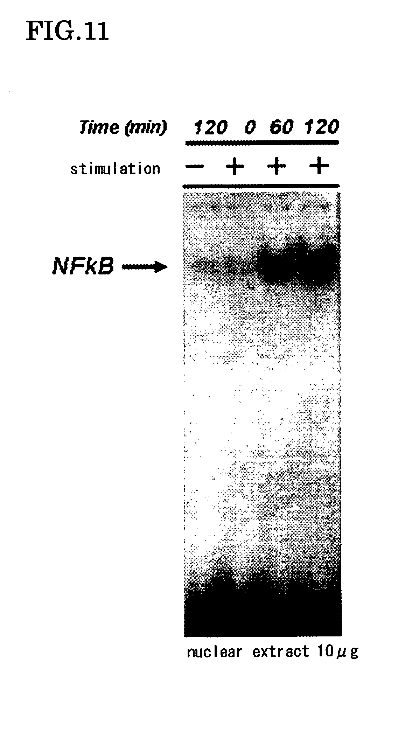

The nuclear extract (control example) prepared from HeLa cell culture by the above process, a HeLa cell nuclear extract immediately after treatment of 2.5 mg/ml of lipopolysaccharide, a HeLa cell nuclear extract 60 minutes after treatment of lipopolysaccharide, and a HeLa cell nuclear extract 120 minutes after treatment of lipopolysaccharide were subjected to a gel shift assay, by using a synthetic oligonucleotide with NFκB binding sequence as a probe. The assay results are shown in FIG. 11.

Concerning the control example (indicated by "-" in FIG. 11) and the example immediately after the treatment (indicated by "time 0" in FIG. 11), NFκB band was not recognized. However, concerning the example 60 minutes after the treatment, and the example 120 minutes after the treatment, bands with a high density corresponding to NFκB were recognized. This shows that NFκB was transferred into the nuclei by treatment of lipopolysaccharide.

The above results show that the amount of an activated transcriptional factor can be determined by measuring the amount of the transcriptional factor in the nuclear extract.

### [Verification on specificity of nucleotide probe]

### (1) Confirmation by HeLa cells

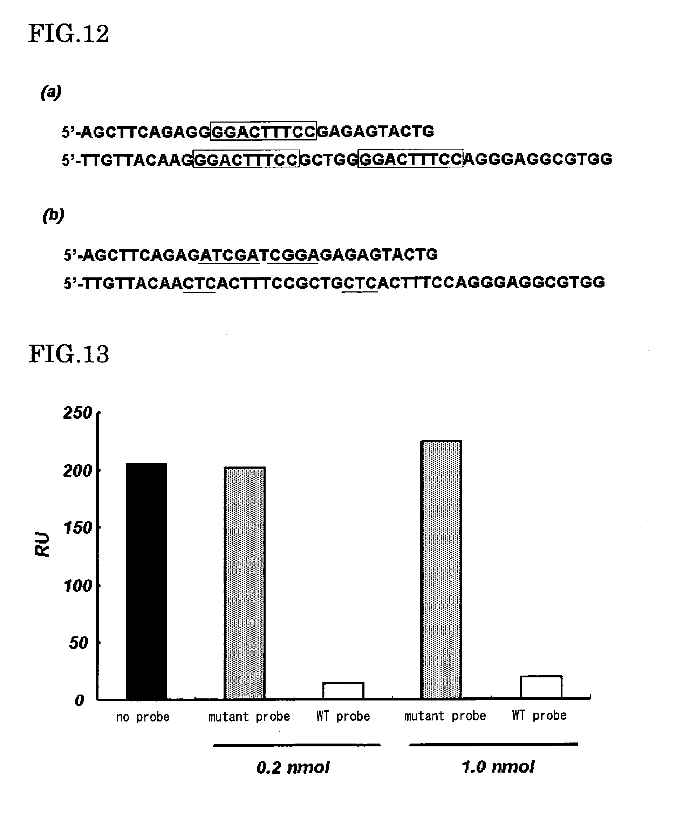

A wild type (WT) oligonucleotide (the portions enclosed by the rectangles is a site to which a transcriptional factor binds) having the sequence as shown in FIG. 12(a), and a mutant probe (the site where mutation is observed is shown by the underlined portions) having the sequence as shown in FIG. 12(b) were used.

120 minutes after treating 2.5 mg/ml of lipopolysaccharide into HeLa cell culture, a nuclear extract was prepared according to the aforementioned process for preparing the nuclear extract sample.

Measurement was conducted by supplying nuclear extracts (0.2 nmol and 1.0 nmol) to an SPR device in respective conditions that a metallic membrane implanted with the WT probe, and a metallic membrane implanted with the mutant probe were used. As a control example, measurement was conducted by supplying a nuclear extract to the SPR device in a condition that a metallic membrane without implantation of a probe was used. The measurement results are shown in FIG. 13.

In FIG. 13, the axis of ordinate shows resonance unit (RU) concerning a complex of a transcriptional factor and a probe. Concerning the respective examples supplied with 0.2 nmol nuclear extract and 1.0 nmol nuclear extract, these examples showed substantially the same level of RU as the control example in case of using the mutant probe, but showed a considerably lower level of RU than the control example in case of using the WT probe. This shows that NFκB contained in the nuclear extract does not bind to the mutant probe, but binds to the WT probe.

### (2) Confirmation by lymphocytes of healthy individuals

Nuclear extracts were prepared according to the aforementioned process for preparing the nuclear extract sample by treating lipopolysaccharide into human lymphocytes of healthy individuals.

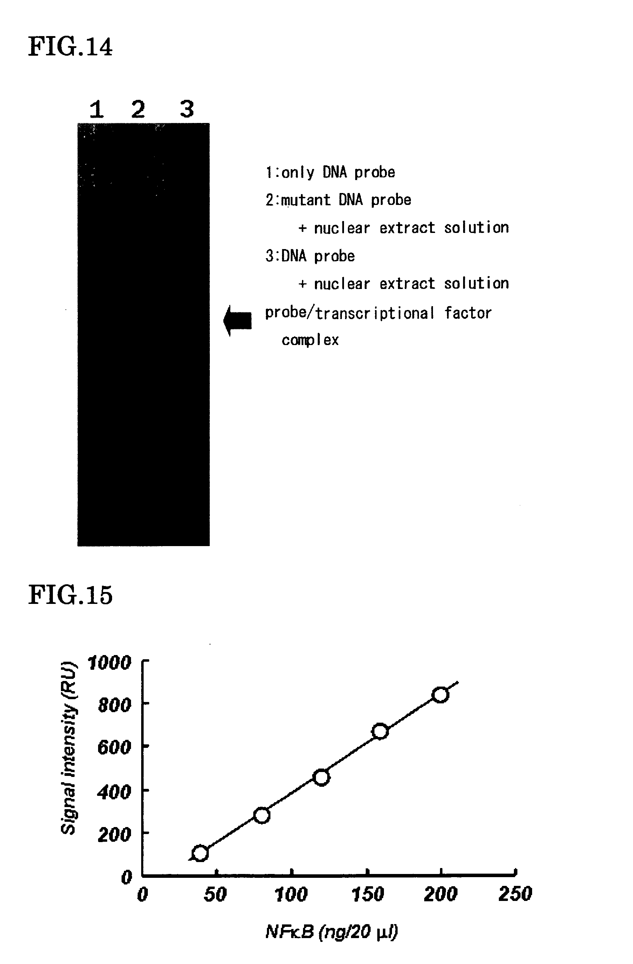

A gel shift assay was conducted, by using the synthetic oligonucleotide probe having NFκB binding sequence or the mutant probe shown in FIG. 12. The assay results are shown in FIG. 14.

In FIG. 14, "1" shows a case that the synthetic oligonucleotide probe having NFκB binding sequence was used, and the nuclear extract was not supplied, "2" shows a case that the mutant probe was used, and the nuclear extract was supplied, and "3" shows a case that the WT probe was used, and the nuclear extract was supplied. The complex of probe and NFκB was intensively recognized in the case "3". The results show that the WT probe is useful as the detection probe of NFκB with respect to healthy individuals, as well as HeLa cells.

### [Detection/measurement of transcriptional factor: NFκB]

Prepared were sample solutions for measurement, respectively containing 40 ng, 80 ng, 120 ng, 160 ng, and 200 ng of NFκB in 20 µl aqueous solution containing 10 mM of HEPES (pH 7.4), 150 mM of NaCl, 3 mM of EDTA, and 0.005% of Tween 20.

The sample solutions were measured by an SPR device (Biacore, BIACORE-J) loaded with the WT probe. The measurement results are shown in FIG. 15. The time required for the respective measurements was 15 minutes. The signal intensity was increased in proportional to the content concentration of NFκB.

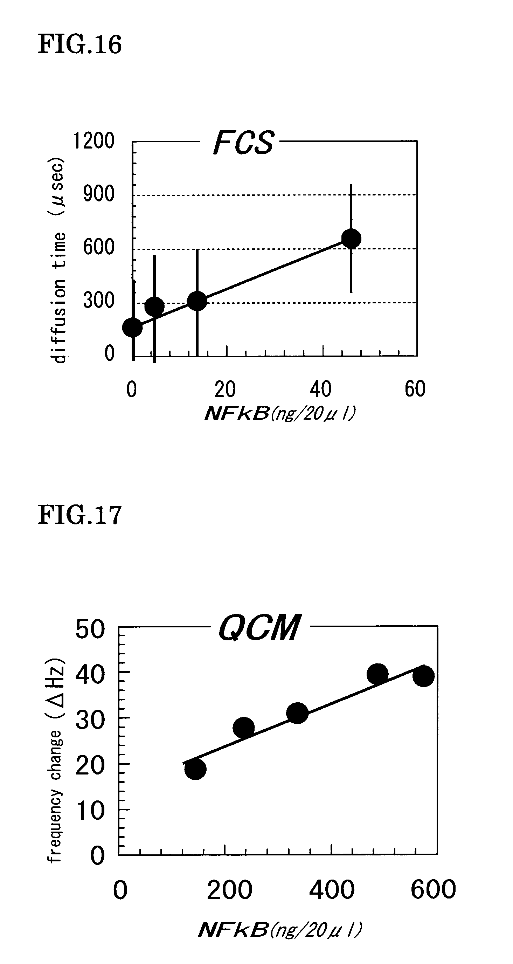

Measurements were conducted concerning the sample solutions in a similar manner as mentioned above, with use of an FCS device (Hamamatsu Photonics K.K.) loaded with the WT probe, and a QCM device (Initiam, Affinix-Q4) loaded with the WT probe. The measurement results are shown in FIGS. 16 and 17, respectively. In both of the measuring methods, the diffusion time or the change in the frequency corresponding to the signal intensity was increased in proportional to the content concentration of NFκB. The time required for the respective measurements was 10 minutes.

In both of the measuring methods, the time required from the point of time when the measurement of NFκB was started until the point of time when the measurement result was obtained is 15 minutes or less. Accordingly, with use of these methods, it is possible to predict the cytokine to be expressed within a few hours before the cytokine is actually secreted from the cells.

### [Detection/measurement of transcriptional factor: SP-1]

Prepared were sample solutions for measurement, respectively containing 215 ng, 430 ng, 645 ng, and 1075 ng of SP-1 in 110 µl aqueous solution containing 10 mM of HEPES (pH 7.4), 150 mM of NaCl, 3 mM of EDTA, and 0.005% of Tween 20.

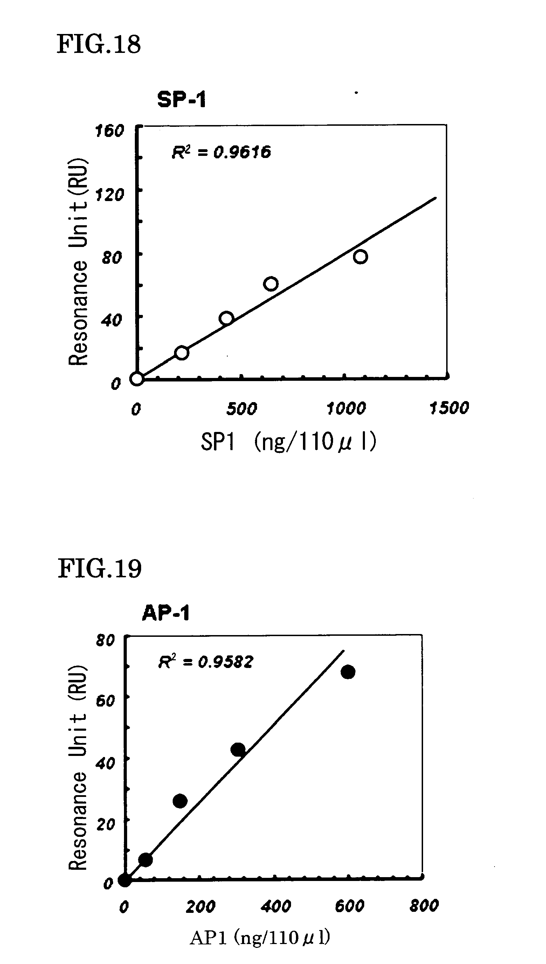

The sample solutions were measured in a similar manner as the measurement on NFκB by the SPR device (Biacore, BIACORE-J), using a metallic membrane implanted with WT probe (5'-TGTATCCCCACCCCCTTAAGAA) and a metallic membrane implanted with mutant probe (5'-TGTATCCCCAAACCCTTAAGAA), respectively. The measurement results are shown in FIG. 18.

As a result of the measurements, it was confirmed that SP-1 in the sample solutions binds to the WT probe but does not bind to the mutant probe. The time required for the respective measurements was 15 minutes. Further, as shown in FIG. 18, the signal intensity (resonance unit) was increased in proportional to the content concentration of SP-1.

### [Detection/measurement of transcriptional factor: AP-1]

Prepared were sample solutions for measurement, respectively containing 60ng, 150ng, 300ng, and 600ng of AP-1 in 110 µl aqueous solution containing 10 mM of HEPES (pH7.4), 150 mM of NaCl, 3 mM of EDTA, and 0.005% of Tween 20.

Each of the sample solutions was measured in a similar manner as the measurement of NFκB, by SPR device (Biacore, BIACORE-J) using a metallic membrane implanted with WT probe (5'-TCGACGTGACTCAGCGCGCATCGTGACTCAGCGCGC) and a metallic membrane implanted with mutant probe (5'-TCGACGTGACTTGGCGCGCATCGTGACTTGGCGCGC), respectively. The measurement results are shown in FIG.19.

It is confirmed that AP-1 in the sample solutions binds to the WT probe but does not bind to the mutant probe. The time required for the respective measurements was 15 minutes. Further, as shown in FIG.18, the signal intensity (resonance unit) was increased in proportional to the content concentration of AP-1.

### [Analysis on cytokine expression, using fibrocytes derived from joint fluids of rheumatic patients]

### (1) Fibrocytes derived from joint fluids of rheumatic patients

After informed consent of rheumatic (RA) patients was obtained, 10 to 15 ml of joint fluids from the RA patients were recovered, and washed with a PBS solution three times. Then, the joint fluids were cultivated in Dulbecco's modified MEM (DMEM) containing 10% fetal calf serum (FCS) at 37 °C under 5% CO₂ condition. Subculture was repeated 5 to 8 times, and the resultant was used in the following experiment.

### (2) Cytokine secretion by thrombin stimulation

The RA-patient-derived fibrocytes (2x10⁵ cells/well) obtained in (1) were cultured in low serum condition (0.5% FCS) for 48 hours before stimulation. Thrombin (10 units/ml) was added to the suspension cell culture. Supernatants were saved from the cell culture immediately (0 time) after thrombin addition, 4 hours, 8 hours, 12 hours, and 24 hours after the addition. The amounts of cytokines (G-CSF, IL2, IL6) secreted by the thrombin stimulation were measured. The cytokines (G-CSF, IL2, IL6) in the cultures were measured, using the respective ELISA kits (Biosource, Camarillo, Calif.). The measurement results are shown in Table 1.

**[Table 1]**

| *Cytokine (pg*/*ml)* | *Time (hr)* | | | | |
|---|---|---|---|---|---|
| | *0* | *4* | *8* | *12* | *24* |
| *IL-6* | *14* ± *5.4* | *16* ± *6.2* | *108* ± *28.5** | *178* ± *34.7** | *288* ± *48.5** |
| *G-CSF* | *10* ± *4.3* | *14* ± *5.5* | *20* ± *7.5* | *39* ± *5.5** | *58* ± *8.8** |
| *IL-2* | *32* ± *8.7* | *26* ± *6.7* | *40* ± *8.3* | *37* ± *7.5* | *42* ± *9.5* |

| | | | | | |
|---|---|---|---|---|---|
| *p<0.05 | | | | | |

As is obvious from Table 1, among the cytokines that have been secreted by the thrombin stimulation in the culture of the fibrocytes from the joint fluids of RA patients, secretions of IL6 and G-CSF were significantly increased 8 hours and 12 hours after the stimulation, respectively. However, significant secretion increase by the thrombin stimulation was not recognized concerning IL2, which is known to belong to the same species as the inflammatory cytokines such as IL6 and G-CSF.

### [Analysis on cytokine mRNA expression using thrombin stimulation]

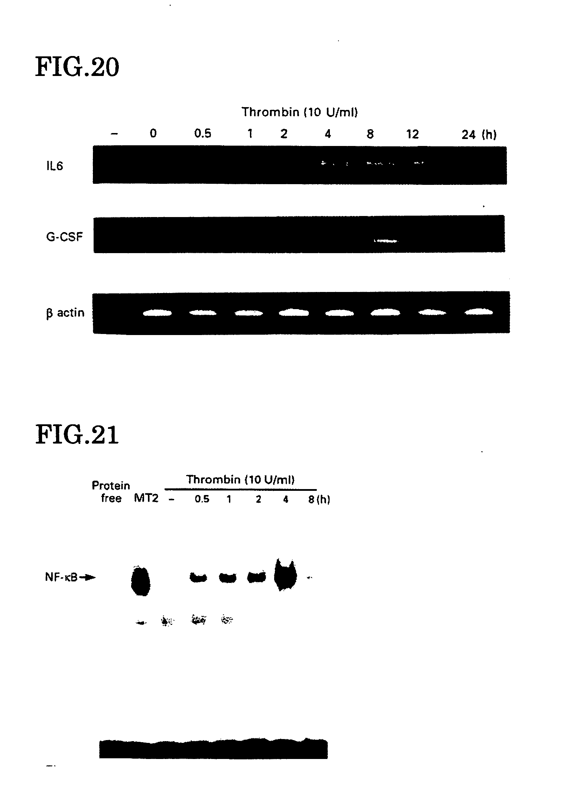

2 x 10⁵ cells/well of fibrocytes derived from RA patient was cultured in a medium containing low serum condition (0.5% FCS) for 48hours before stimulation. Thrombin (10 units/ml) was added to the suspension cell culture. Cells were collected immediately after thrombin addition, and thereafter, at 0.5 hours, 1 hour, 2 hours, 4 hours, 8 hours, 12 hours, and 24 hours after the thrombin addition, and RNA was extracted from the collected cells each time. Reverse transcriptase was added to the obtained RNA samples and cDNA was synthesized. The cDNA was amplified by PCR using primers for measurement of the cytokines shown below. And then, the PCR product was loaded to agarose gel electrophoresis, and the target mRNAs were detected and measured. The measurement results are shown in FIG.20. In FIG.20, β actin was used as a control.

Primers used for RT-PCR method are as follows:
IL6-1; 5'-GCGCCTTCGGTCCAGTTGCCTTGTC
IL6-2; 5'-CCTCTTTGCTGCTTTCACACATG
G-CSF-1; 5'-ACAGTGCACTCTGGACAGT
G-CSF-2; 5'-TCCAGCTGCAGTGTGTCCA

As shown in FIG.20, in the case of IL6, mRNA encoding IL6 was detected at 4 hours after stimulation. In the case of G-CSF, mRNA encoding G-CSF was detected at 4 to 8 hours after stimulation.

### (4) measurement of active NFκB using thrombin stimulation

### (4-1) preparation of nuclear extract

2x 10⁵ cells/well of fibrocytes derived from RA patient used in (1) was cultured in a medium containing low serum condition (0.5% FCS) for 48 hours before stimulation. Thrombin (10 units/ml) was added to the suspension cell culture. Cells were collected from the suspension immediately after the addition. The collected cells were washed with PBS, centrifuged, and solubilized by adding hypotonic solution (10 mM HEPES/KOH (pH7.8), 10mM KC1, 0.1mM EDTA, 1mM DTT, 0.1% NP-40 and protease inhibitor (Sigma)) and reacting for 30 minutes under cooling with ice. The resultant solution was centrifuged at 4 °C, 2500×g for 1 minute, and then, precipitate (fraction containing nuclei) was obtained. A solution (50 mM HEPES/KOH(pH7.8), 0.1 mM EDTA, 5 mM MgCl₂, 2% glycerol, 1 mM DTT, 0.42 M KCl, and protease inhibitor(Sigma)) was added to the nuclei fraction and solubilize the nuclei contained in the fraction. The nuclei-solubilized solution was centrifuged at 4 °C, 12000×g for 15 minutes, the supernatant were saved as the nuclear extract.

### (4-2) Gel shift assay

The nuclear extract (with adequate concentration) prepared in (4-1), 1 µg/ml of Poly-dl/dC, and 20 fmol of FITC labeling probe (5'-FITC-AGTTGAGGGACTTTCCCAGGC) (custom-made probe by Invitrogen Japan K.K.) were added to a solution containing 10 mM of Tris/HCL (pH 7.1), 50 mM of KCl, 1 mM of DTT, 5% of glycerol, 0.25% of NP-40, and 1 mM of MgCl₂, and the resultant was reacted at 4 °C for 30 minutes. After the reaction, adequate amounts of 25% glycerol and a blue dye (bromophenol blue) were added, and electrophoresis (150V, 4 °C, 2 to 4 hours) was conducted in 5% polyacrylamide gel which had been energized (150V, 90 minutes). After the electrophoresis, the fluorescent dye in the gel was visualized using a molecular imager FX (Bio Rad) for analysis. The analysis results are shown in FIG. 21.

As is obvious from FIG. 21, the amount of active NFκB was increased 30 minutes after the thrombin stimulation, and reached a peak 4 hours after the stimulation.

As shown in the results concerning cytokine secretion by thrombin stimulation (Table 1), expression of mRNA (FIG. 20), and gel shift assay (FIG. 21), it is conceived that the expression of IL6 and G-CSF is controlled on DNA by an increase in the amount of active NFκB. On the other hand, IL2 is a cytokine coded by DNA without NFκB binding sequence. Therefore, an increase in the mRNA expression level and the secretion amount of IL2 was not recognized, despite the increase of the amount of active NFκB by thrombin stimulation. Conceivably, these results show the following: expression of IL6 and G-CSF is increased in the fibrocytes derived from the joint fluids of RA patients (chronic inflammation patients), as the amount of active NFκB is increased due to a physiologically active substance or an inflammation causing substance, and expression of IL2 coded by DNA without NFκB binding sequence is controlled by an independent transcriptional factor sequence (NFAT, AP-1). Accordingly, in predicting as to whether a symptom resulting from expression of IL6 or G-CSF will occur concerning RA patients, measurement of the amount of NFκB in nucleus (amount of active NFκB) may be conducted with respect to biopsy tissue samples from the patients. The NFκB is a transcriptional factor commonly involved in expression of the cytokines IL6 and G-CSF. Therefore, it is concluded that information as to whether an inflammatory response is increased can be obtained by the measurement.

### EXPLOITATION IN INDUSTRY

According to the inventive diagnosis support method, information based on a prediction result concerning a cytokine to be expressed within a few hours can be obtained. This enables to predict a symptom which is supposed to occur within a few hours for selecting a treatment method, concerning diseases whose symptoms are changed with time, i.e. immune system malfunction diseases such as sepsis or SIRS, whose proper treatment has been difficult by a conventional diagnosis method such as a diagnosis method based on symptoms or a method of measuring the amount of a cytokine in a sample obtained from a patient.

Accordingly, by installing the inventive diagnosis support information output device in a medical site, selection of a suitable treatment method for the symptom which is supposed to occur within a few hours is feasible, concerning the immune system malfunction diseases, of which accurate knowledge on the cause of the symptom was difficult to obtain by the conventional art. Thus, the inventive method is advantageous in increasing the survival rate, and shortening the stay time in ICU.

## Claims

1. A supporting method for diagnosing immune system malfunction disease, the method comprising:
measuring an amount of an activated transcriptional factor specific to a cytokine selected from the group consisting of inflammatory cytokines and anti-inflammatory cytokines in a biological sample obtained from a patient; and
outputting diagnosis support information including information relating to progress condition of inflammations in the future, based on the measured amount of the activated transcriptional factor.

2. A diagnosis supporting method according to claim 1, wherein the information relating to progress condition of inflammation is selected from the group consisting of information as to whether an inflammatory response will be increased, and information as to whether an anti-inflammatory response will be increased.

3. A diagnosis supporting method according to claim 1, wherein the information relating to progress condition of inflammation is selected from the group consisting of information as to whether the expression level of an inflammatory cytokine is increased, and information as to whether the expression level of an anti-inflammatory cytokine is increased.

4. A diagnosis supporting method according to claim 1, wherein the diagnosis support information comprises an information relating to treatment method of the disease.

5. A diagnosis supporting method according to claim 4, wherein the treatment method is selected from the group consisting of administration of an anti-inflammatory agent, exchange of plasma, and administration of granulocyte colony-stimulating factor.

6. A diagnosis supporting method according to claim 1, wherein the sample is nuclear extract of cells obtained from the patient.

7. A diagnosis supporting method according to claim 1, wherein the measuring step is performed with use of a measuring method not requiring more than 30 minutes for a time from a measurement start until a measurement result is obtained.

8. A diagnosis supporting method according to claim 7, wherein the measuring method is one selected from the group consisting of surface plasmon resonance method, quartz crystal microbalance method, and fluorescence correlation spectroscopy method.

9. A diagnosis supporting method according to claim 1, wherein the measuring step is conducted by measuring two or more kinds of activated transcriptional factors.

10. A diagnosis supporting method according to claim 1, wherein the immune system malfunction disease includes systemic inflammatory response syndrome (SIRS).

11. A diagnosis support information output device concerning immune system malfunction diseases, the device comprising:
an information acquiring means for acquiring information relating to transcriptional factors obtained by measuring an amount of an activated transcriptional factor specific to a cytokine selected from the group consisting of inflammatory cytokines and anti-inflammatory cytokines, in the biological sample obtained from a patient;
a diagnosis support information outputting means for outputting diagnosis support information including information relating to progress conditions of inflammations in the future, based on the acquired regulator information.

12. A diagnosis support information output device according to claim 11, wherein the information relating to progress condition of inflammation is selected from the group consisting of information as to whether an inflammatory response will be increased, and information as to whether an anti-inflammatory response will be increased.

13. A diagnosis support information output device according to claim 11, wherein the information relating to progress condition of inflammation is selected from the group consisting of information as to whether the expression level of an inflammatory cytokine is increased, and information as to whether the expression level of an anti-inflammatory cytokine is increased.

14. A diagnosis support information output device according to claim 11, wherein the diagnosis support information comprises information relating to treatment method of the disease.

15. A diagnosis supporting method according to claim 14, wherein the treatment method is selected from the group consisting of administration of an anti-inflammatory agent, exchange of plasma, and administration of granulocyte colony-stimulating factor.

16. A diagnosis support information output device according to claim 11, wherein the diagnosis support information outputting means includes a storing means for storing information relating to a transcriptional factor specific to an inflammatory cytokine, and a predicting means for predicting whether the expression level of an inflammatory cytokine will be increased, based on the information stored in the storing means and the transcriptional factor information acquired by the information acquiring means, wherein diagnosis support information is outputted based on a prediction result by the predicting means.

17. A diagnosis support information output device according to claim 16, wherein the storing means further stores information relating to a transcriptional factor specific to an anti-inflammatory cytokine.

18. A diagnosis support information output device according to claim 11, wherein the diagnosis support information outputting means includes a storing means for storing information as to whether an inflammatory response is increased, and a predicting means for predicting whether an inflammatory response will be increased, based on the information stored in the storing means and the transcriptional factor information acquired by the information acquiring means, wherein diagnosis support information is outputted based on a prediction result by the predicting means.

19. A diagnosis support information output device according to claim 18, wherein the storing means further stores information as to whether an anti-inflammatory response will be increased.
